# EUROPEAN PATENT APPLICATION

(11) **EP 2 910 940 A1**
(43) Date of publication of application: **26.08.2015**
(21) Application number: 14000587.7
(22) Date of filing: 19.02.2014
(51) Int. Cl.: G01N 27/12, G01N 33/00

(54) **Composition of a poly ionic liquid and a metal compound for the detection of CO2 gas at low temperatures**

(71) Applicant: ETH Zurich, 8092 Zurich (CH)
(72) Inventor: Niederberger, Markus, 6386 Wolfenschiessen (CH); Kränzlin, Niklaus, 8003 Zürich (CH); Koziej, Dorota, 8005 Zürich (CH)

(57) **Abstract**

Composites of poly ionic liquids and metal compounds for low temperature CO₂ gas detecting, a solution or a suspension comprising said composition, a substrate comprising said composition, a use of said composition for CO₂ detection or in a CO₂ sensor or a use of said suspension or solution in a method for manufacturing a CO₂ sensor. Further comprised is a CO₂ sensor comprising said composition and a method for detecting CO₂.

## Description

### Field of the invention

The present invention relates to composites of poly ionic liquids and metal compounds in particular for low temperature CO₂ gas detecting, a solution or a suspension comprising said composition, a substrate comprising said composition, a use of said composition for CO₂ detection or in a CO₂ sensor or a use of said suspension or solution in a method for manufacturing a CO₂ sensor. The invention further comprises a CO₂ sensor comprising said composition and a method for detecting CO₂.

### Background of the invention

Miniaturization is one of the main issues but also a driving force in the research development as well as fabrication of electronic devices. This is also valid for CO₂ sensors, which are used in a wide extent of applications ranging from air quality monitoring in motors and in buildings to factories of the beverage industry. Additionally, to those established markets, the need for control of carbon dioxide concentration in breath for instance of a scuba diver, Himalaya climber or emphysema patient is driving the development of CO₂ sensors that are portable.

However, the results obtained so far with metal oxides-based sensors are disappointing, particularly when compared to advanced and commercialized sensors for reducing or oxidizing gases. Intensive trail-and-error work is under way, but the prospect of simple-in-use CO₂ sensors appears remote. The realization of a CO₂ sensor is very challenging and depends on the combination of new structural materials that deliver active properties and simultaneously maintain a simple transduction scheme.

So far, most CO₂ sensors are based on the non-dispersive infrared method (NDIR).

Their sensitivity depends on the length of the absorption optical path path (Lambert-Beer equation). Thus, the aforementioned miniaturization reaches its limits.

Thus, a new type of CO₂ sensing based on a different technology is being needed.

There is an increasing interest towards CO₂ sensors based on the chemo-resistive effect. These sensors show an increase or decrease of resistance when subjected to

CO₂.The advantages mainly lie in the fact that they can be miniaturized, having moderate production costs.

Recently, the sensitivity of oxycarbonates of lanthanum and neodymium to CO₂ was reported (A. Haensch, D. Koziej, M. Niederberger, N. Barsan, U. Weimar, Procedia Engineering 2010, 5, 139). The document describes an increase of the resistance of the oxycarbonate film with increasing concentration of CO₂.

However, to date, most chemo-resistive CO₂ sensors work at high temperatures (application temperatures between 200 and 700 °C), thus, negatively affecting long term stability of these sensors. Also, these sensors are heated resistively, thus, energy is consumed. Furthermore, the use of conductive polymers show in CO₂ sensor resistance changes upon exposure to CO₂. The most studied are the polypyrrols and their composites with metal oxides (see Waghuley, S. A; Yenorkar, S. M.; Yawale, S. S.; Yawale, S. P. Application of chemically synthesized conducting polymer-polypyrrole as a carbon dioxide gas sensor Sens. Actuators, B 2008,128, (2), 366-373).

The problem of the invention is to develop a composition usable in a CO₂ sensor working at ambient temperature, which can be easily miniaturized and has moderate production costs and has no cross-sensitivity to reducing or oxidizing gases like CO, H₂ or NOₓ (mono-nitrogen oxides).

This problem is solved by a composition according to claim 1, a suspension or solution according to claim 8, a substrate according to claim 11 or a CO₂ sensor according to claim 15.

### Summary of the invention

According to a first aspect of the invention, the invention relates to a composition, in particular a composition for CO₂ detecting, comprising at least one metal compound MC, in particular a rare earth compound REC, more particularly a rare earth oxycarbonate REOC, and a poly ionic liquid PIL.

According to a second aspect of the invention, the invention relates to a suspension or solution comprising a composition according to the first aspect of the invention.

According to a third aspect of the invention, the invention relates to a substrate comprising a composition according to the first aspect of the invention, wherein on said substrate said at least one metal compound MC, in particular said at least one rare earth compound REC, more particularly said at least one rare earth oxycarbonate REOC and said poly ionic liquid PIL are deployed, wherein in particular said substrate is selected from a rigid material, such as ceramic, alumina (Al₂O₃), sapphire, silicium or glass or flexible material such as polyamide or polyurethane.

According to a fourth aspect of the invention, the invention relates to the use of the composition according to the first aspect of the invention or the use of a substrate according to the third aspect of the invention for CO₂ detection.

According to a fifth aspect of the invention, the invention relates to the use of a composition according to the first aspect of the invention or the use of substrate according to the third aspect the invention in a CO₂ sensor.

According to a sixth aspect of the invention, the invention relates to the use of a suspension or solution according to the second aspect of the invention in screen printing, drop casting, tape casting, spin casting or ink jet printing methods, in particular in screen printing, drop casting, tape casting, spin casting or ink jet printing methods for manufacturing a CO₂ sensor.

According to a seventh aspect of the invention, the invention relates to a CO₂ sensor comprising a composition according to the first aspect of the invention or a substrate according to the third aspect of the invention or a CO₂ sensor manufactured by use of the suspension or solution according to the second aspect of the invention.

According to an eight aspect of the invention, the invention relates to a method for CO₂ detection comprising a CO₂ sensor according to the seventh aspect of the invention.

As used herein the term "metal compound," refers to a compound comprising a metal element and a non-metal moiety ("counterpart") such as an oxide, a hydroxide, a carbonate or an oxycarbonate and the mixture of thereof. The metal compound may comprise a covalent or ionic structure.

As used herein the term "actinoides" refers to the metallic chemical elements with atomic numbers from 89 to 103 (actinium to lawrencium).

As used herein the term "lanthanoides" refers to the metallic chemical elements with atomic numbers from 57 to 71 (lanthanum to lutetium).

As used herein the term "rare earth metal" refers to the lanthanoides plus scandium and yttrium.

### Detailed description of the invention

The first aspect of the invention relates to a composition, in particular for CO₂ detection, comprising a metal compound MC and poly ionic liquid PIL.

In some embodiments, the metal compound MC comprises chemo-resistive properties in particular chemo-resistive properties selective to CO₂. Compositions comprising chemo-resistive properties comprise an increase or decrease of resistant when subjected to a gas or a gas-mixture. The contact with the gas or the gas-mixture has a direct influence on the conductivity of the material and, thus, leading to a change in the resistance, which can be measured. The change of the resistance correlates with the concentration of the gas or gas-mixture, which is to be measured.

In some embodiments, the metal compound MC comprises chemo-capacitive properties in particular chemo-capacitive properties selective to CO₂. Compositions comprising chemo-capacitive properties comprise an increase or decrease of capacitance when subjected to a gas or a gas-mixture. The contact with the gas or the gas-mixture has a direct influence on the capacity of the material and, thus, leading to a change in the capacitance, which can be measured. The change of the capacitance correlates with the concentration of the gas or gas-mixture, which is to be measured

In some embodiments, the metal of said metal compound MC is selected from rare earth metals, metals from the group of lanthanides or metals from the group of actinoides, and/or the metal compound MC is selected from a metal hydroxide, metal carbonate, metal oxide or metal oxycarbonate or mixture thereof.

Thus, said metal compound MC may comprise at least two metal compounds MC, wherein the metal compounds MC may comprise the same metal but different counterparts such as oxide or oxycarbonate (e.g. a lanthanum oxide and a lanthanum oxycarbonate). Said metal compound MC may also comprise at least two metal compounds MC, wherein the metals may be different but the counterparts are the same (e.g. a lanthanum oxide and a cerium oxide). Furthermore, said metal compound MC may comprise at least two metal compounds MC, wherein the metals and the counterparts may be different (e.g. a lanthanum oxycarbonate and a cerium oxide).

In some embodiments, the metal compound MC is selected from a metal oxide or a metal oxycarbonate.

In some embodiments, the metal compound MC is a metal oxycarbonate.

In some embodiments, said metal compound MC is a rare earth compound REC.

In some embodiments, the metal of said metal compound MC is a rare earth metal REC, wherein said rare earth metal REC is selected from a rare earth metal hydroxide, a rare earth metal carbonate, a rare earth metal oxide or a rare earth metal oxycarbonate, in particular from a rare earth metal oxide or a rare earth metal oxycarbonate, more particularly a rare earth metal oxycarbonate.

In some embodiments, said metal compound MC is a rare earth compound REC, wherein the rare earth metal of said rare earth compound REC is selected from lanthanum, cerium, neodymium, praseodymium or gadolinium.

In some embodiments, said metal compound MC is a rare earth compound REC, wherein the rare earth metal of said rare earth compound REC is selected from lanthanum, cerium, neodymium or praseodymium.

In some embodiments, said metal MC is a rare earth compound REC, wherein the rare earth metal of said rare earth compound REC is lanthanum.

In some embodiments, said metal compound MC is a rare earth oxycarbonate REOC.

In some embodiments, said metal compound MC is a rare earth oxycarbonate REOC, wherein the rare earth metal of said rare earth oxycarbonate REOC is selected from lanthanum, cerium, neodymium, praseodymium or gadolinium.

In some embodiments, said metal compound MC is a rare earth oxycarbonate REOC, wherein the rare earth metal of said rare earth oxycarbonate REOC is selected from lanthanum, cerium, neodymium or praseodymium.

In some embodiments, said metal compound MC is a rare earth oxycarbonate REOC, wherein the rare earth metal of said rare earth oxycarbonate REOC is lanthanum.

In some embodiments, said metal compound MC is a rare earth oxycarbonate REOC.

The term "PIL" refer to a special type of polyelectrolates which carry an ionic liquid species in each of the repeating unit. They combine properties of ionic liquids and the flexibility of polymers.

In some embodiments, the poly ionic liquid PIL comprises a, in particular reversible, CO₂ capturing capacity.

In some embodiments, the poly ionic liquid PIL is selected from polymers of pyrrolidinium based ionic liquids, sulfonium based ionic liquids, alanines based ionic liquids, cholines based ionic liquids, acetonitrile based ionic liquids, ammonium based ionic liquids, pyridinium based ionic liquids, imidazolium based ionic liquids or phosphonium ionic liquids or the poly ionic liquid PIL comprises a mixture of the before mentioned.

In some embodiments, the poly ionic liquid PIL is selected from polymers of ammonium based ionic liquids, pyridinium based ionic liquids, imidazolium based ionic liquids or phosphonium ionic liquids.

In some embodiments, the poly ionic liquid PIL is selected from polymers of ammonium based ionic liquids. Tetraalkylammonium-based PIL show superior CO₂ sorption capacities, thus, these PIL are preferred candidates for application in CO₂ gas sensors.

In some embodiments, the poly ionic liquid PIL is selected from p-vinylbenzyl)triethyl ammonium tetrafluoroborate P[VBTEA][BF₄], 1-(p-vinylbenzyl)pyridinium tetrafluoroborate P[VBP][BF₄], (p-vinylbenzyl)trimethyl ammonium hexafluorophosphate P[VBTMA][PF₆], 2-(methacryloyloxy) ethyltrimethylamnonium tetrafluoroborate P(MATMA][BF₄], (p-vinylbenzyl)trimethyl ammonium trifluoromethane sulfonamide P[VBTMA][Tf₂N]; p-vinylbenzyl)trimethyl ammonium tetrafluoroborate P[VBTMA][BF₄], 1-(p-vinylbenzyl)-3-methyl-imidazolium tetrafluoroborate P[VBMI][BF₄], from (p-vinyl benzyl)tributylammonium tetrafluoroborate P[VBTBA][BF₄] or p-vinylbenzyl)triethyl phosphonium tetrafluoroborate P[VBTEP][BF₄], in particular said poly ionic liquid (PIL) is (p-vinylbenzyl)trimethyl ammonium hexafluorophosphate P[VBTMA][PF₆].

In some embodiments, the poly ionic liquid PIL comprises a mixture of the poly ionic liquids PIL defined in the previous paragraph.

In some embodiments, the poly ionic liquid is (P-venylbenzyl) trimethylammoniumhexaflurophosphat P[VBTMA][PF₆].

The poly ionic liquid P[VBTMA][PF₆] has a polystyrene backbone, an inorganic anion and a small substituent on the cation side and a high CO₂ sorption capacity. The poly ionic liquid P[VBTBA][PF₆] has a high sensitivity (it can absorb 77% of its body weight of CO₂); a good selectivity/cross-sensitivity (the CO₂ absorption is very selective since no mass change was observed for N₂, O₂ and CH₄ sorption); a fast response/recover time and stability (at room temperature sorption and desorption is on the order of minutes of up to the 95% of CO₂ capacity and stable over time); a high thermal stability (Tg = 255°C); and is easy to process since it is soluble in acetonitrile.

Poly ionic liquids P[VBTMA][Tf₂N) comprising a mesoporous P[VBTMA][Tf₂N) networks show significantly faster CO₂ adsorption than its bulk counterparts (see Tang, J. B.; Shen, Y. Q.; Radosz, M.; Sun, W. L. Isothermal Carbon Dioxide Sorption in Poly(ionic liquid)s Ind. Eng. Chem. Res. 2009, 48, (20), 9193-9118).

In some embodiments, the compositions comprises as said metal compound MC a rare earth oxycarbonate REOC, wherein in particular the rare earth metal of said rare earth oxycarbonate REOC is selected from lanthanum, cerium, neodymium, praseodymium or gadolinium, in particular from lanthanum, cerium, neodymium or praseodymium, more particularly the rare earth metal of said rare earth oxycarbonate REOC is lanthanum and
- said poly ionic liquid PIL comprises a polymer structure of ionic liquid monomers which comprise a reversible CO₂ capturing capacity, or
- said poly ionic liquid PIL is selected from pyrrolidinium based ionic liquids, sulfonium based ionic liquids, alanines based ionic liquids, cholines based ionic liquids, acetonitrile based ionic liquids, ammonium based ionic liquids, pyridinium based ionic liquids, imidazolium based ionic liquids or phosphonium ionic liquids, or
- said poly ionic liquid PIL is selected from ammonium based ionic liquids, pyridinium based ionic liquids, imidazolium based ionic liquids or phosphonium based ionic liquids, or
- said poly ionic liquid PIL is selected from ammonium based ionic liquids, or
- said poly ionic liquid is selected from p-vinylbenzyl)triethyl ammonium tetrafluoroborate P[VBTEA][BF₄]_{,} 1-(p-vinylbenzyl)pyridinium tetrafluoroborate P[VBP][BF₄], (p-vinylbenzyl)trimethyl ammonium hexafluorophosphate P[VBTMA][PF₆], 2-(methacryloyloxy) ethyltrimethylamnonium tetrafluoroborate P[MATMA][BF₄], (p-vinylbenzyl)trimethyl ammonium trifluoromethane sulfonamide P[VBTMA][Tf₂N]; p-vinylbenzyl)trimethyl ammonium tetrafluoroborate P[VBTMA][BF₄], 1-(p-vinylbenzyl)-3-methyl-imidazolium tetrafluoroborate P[VBMI][BF₄], from (p-vinyl benzyl)tributylammonium tetrafluoroborate P[VBTBA][BF₄] or p-vinylbenzyl)triethyl phosphonium tetrafluoroborate P[VBTEP][BF₄], or
- said poly ionic liquid PIL is (p-vinylbenzyl) trimethylammoniumhexaflurophosphat P[VBTMA][PF₆].

The composition of the invention comprises metal compounds, in particular rare earth compounds, more particular rare earth oxycarbonates, and a poly ionic liquid, which are interacting with each other.

As already discussed, the use of rare earth oxycarbonates or oxides, is known in the art. These salts have some advantages for the use in detecting CO₂, since they comprise a high sensor signal and they are good transduction elements, which can conduct chemical information into an electrical signal. However, a major disadvantage of these compounds is the necessity to use a very high working temperature. CO₂ sensors comprising such compound cannot operate at room temperature and the high working temperature negatively affects long term stability of these sensors and the use of such sensors has a very high demand on energy.

Characteristics of poly ionic liquids PIL are also known in the art. PIL are solid polymeric materials, which are generally prepared via polymerization of either the cation or the anion species of the respective ionic liquid. An ionic liquid on the other hand is composed of two species with opposed charges. PIL have been shown to selectively absorb CO₂ in large amounts.

The use of a composite comprising metal compounds MC, in particular rare earth compounds REC, more particularly rare earth oxycarbonates REOC and poly ionic liquids PIL are not known in the art. Such composites allow the provision of a material for detection of CO₂, which can readily miniaturized and the provision of a CO₂ sensor which may be operated at a temperature range between room temperature and 200 °C. Such a working temperature has a significant advantage in comparison to known CO₂ sensors.

The composition of the invention comprises further an excellent thermal and chemical stability together with a good flexibility.

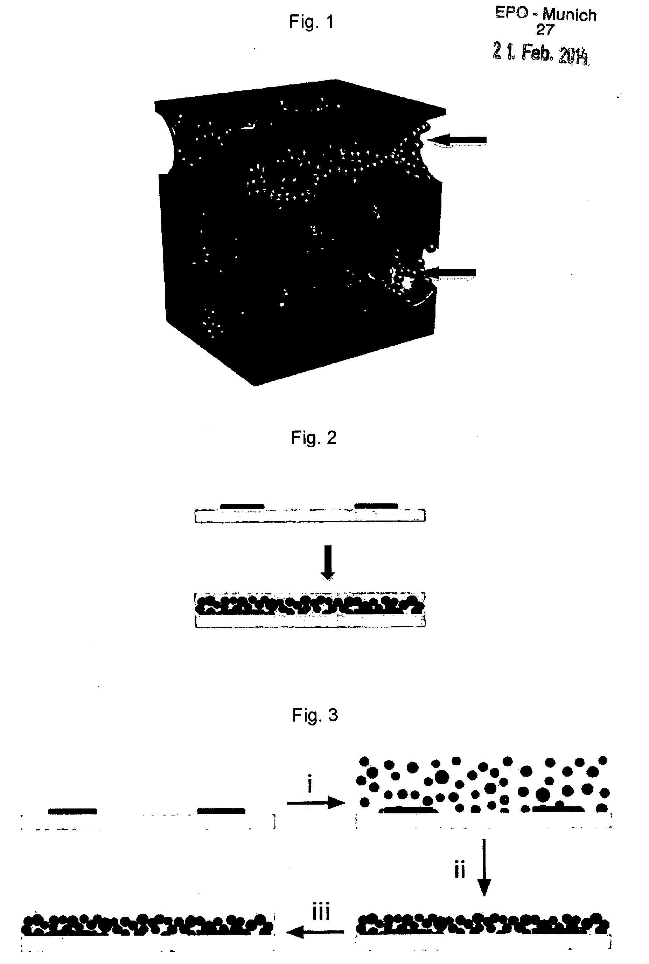

An example of such a structure is depicted in Fig. 1. The poly ionic liquid PIL in a composition according to the invention selectively absorbs CO₂ and increase the probability for a CO₂ interaction with the particles of the metal compound MC, in particular the rare earth compound REC, more particularly the rare earth oxycarbonate REOC. The pre-concentration is crucial for operation at room temperature because it increases the efficiency of the CO₂ interaction.

The metal compound MC, in particular the rare earth compound REC, more particularly the rare earth oxycarbonate REOC of the composition according to the invention works as an electronic transduction element in which the particles are transducing the chemical information into an electric signal.

Thus, the polymeric phase PIL selectively absorbs CO₂ while the metal compound MC, in particular the rare earth compound REC, more particularly the rare earth oxycarbonate REOC, change their electric properties upon contact with CO₂. The CO₂ absorption and adsorption properties of the PIL amplify the reactivity toward CO₂ with the metal compound MC, in particular the rare earth compound REC, more particularly the rare earth oxycarbonate REOC at room temperature.

Furthermore, the most essential is that composition according to the invention comprises a synergistic effect. The inventors believe, without being bound by this theory that the synergistic effect arises from anchoring the poly ionic liquids PIL at the surface of the particles, for example in case of using a rare earth oxycarbonate and an ammonium based PI L, through interaction between strongly electropositive metal ions of the metal compound MC and cations of the PIL. Neither, PI L nor the MC, in particular the REOC-film itself is having measurable resistance (<20 GOhms) at room temperature.

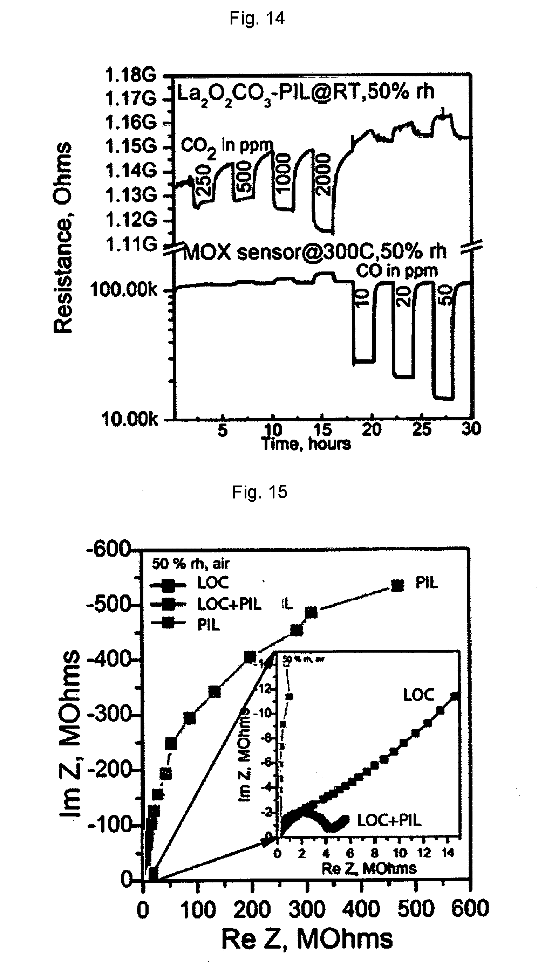

However, at the certain concentrations of PIUMC, in particular PIL/REOC, the resistivity of the films dramatically decreases, as it is revealed by EIS measurements shown in Fig. 15. It has to deal with charge transfer between PIL and the surface of nanoparticles. At a certain percolation level the interaction between the PIL and MC, in particular REOC, are governing the electrical characteristic of the films.

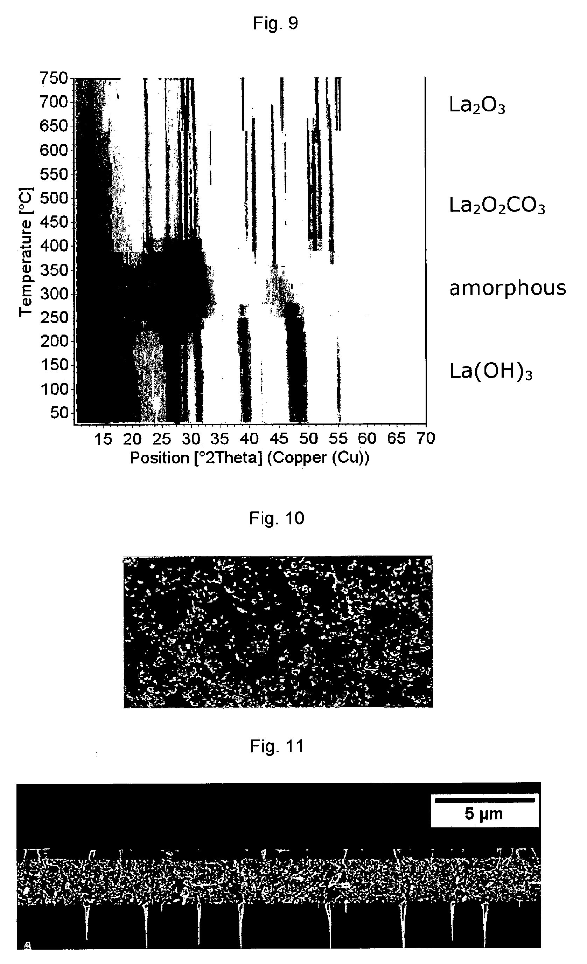

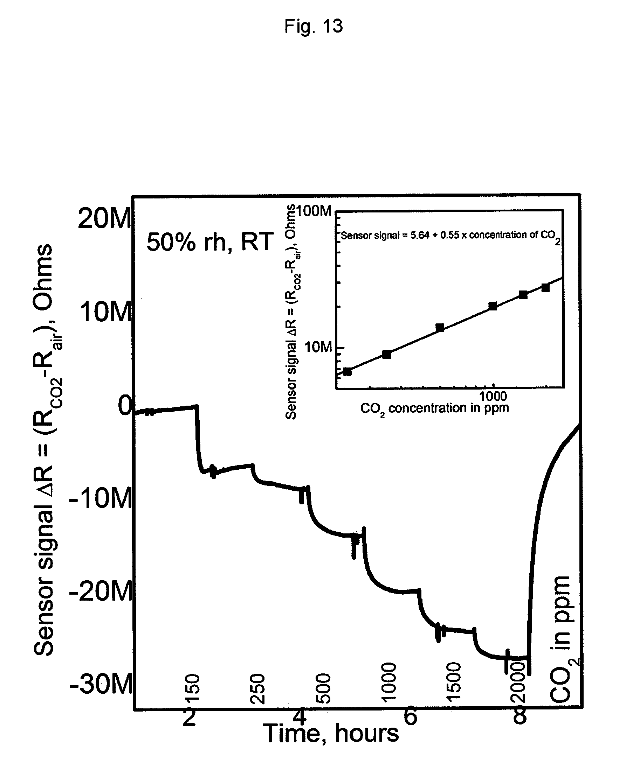

Fig. 10 to 18 show an example of the effect of one composition of the invention, namely lanthanum oxycarbonate (LOC) and P[VBTMA][PF₆]. For this nanocomposite, the chemo-resistive principle was tested providing new insight into this type of sensing. The findings from both resistance and impedance measurements attest the validity to use such composite systems in CO₂ sensing. Moreover, even though the sensors were tested at room temperature, the sensor signal clearly shows sensitivity to CO₂.

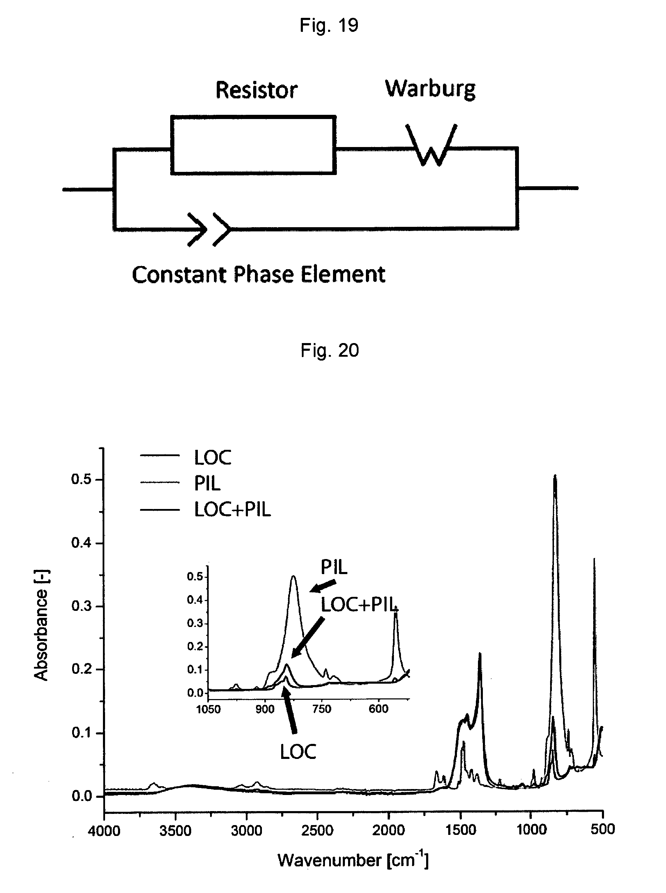

The composition of the invention shows a synergistic effect, which is depicted in Fig. 15 and Fig. 20.

In Fig. 15 the electrical properties of La₂O₂CO₃P[VBTMA][PF₆] composites are shown. The impedance spectroscopy visualized the synergistic influence of La₂O₂CO₃/P[VBTMA)[PF₆] on the electrical properties of the composites by comparing the La₂O₂CO₃/P[VBTMA)[PF₆] composite and its individual components La₂O₂CO₃ and P[VBTMA)[PF₆].

It has to be noted that impedance of the La₂O₂CO₃/P[VBTMA)[PF₆] sensor is lower than that of La₂O₂CO₃ sensor and much lower than the one of the polymer itself. For the composite material the presence of the semicircle at high frequencies (50 Hz - 2 MHz) suggests that in comparison with the individual components, there is an additional charge transfer at the organic/inorganic interface. Furthermore, the organic-inorganic interface formation is lowering of the resistivity also at DC (50 Hz) that makes the resistive readout feasible.

In some embodiments, the ratio of said metal compound MC, in particular said rare earth compound REC, more particularly said rare earth oxycarbonate REOC, to said poly ionic liquid PIL is in the range of 1 wt% to 99 wt% of said metal compound MC, in particular said rare earth compound REC, more particularly said rare earth oxycarbonate REOC, to 99 wt% to 1 wt% of said poly ionic liquid PIL.

In some embodiments, the ratio of said metal compound MC, in particular said rare earth compound REC, more particularly said rare earth oxycarbonate REOC, to said poly ionic liquid PIL is in the range of 80 wt% to 20 wt% of said of said metal compound MC, in particular said rare earth compound REC, more particularly said rare earth oxycarbonate REOC, to 20 wt% to 80 wt% of said poly ionic liquid PIL.

In some embodiments, the ratio of said metal compound MC, in particular said rare earth compound REC, more particularly said rare earth oxycarbonate REOC, to said poly ionic liquid PIL is in the range of 80 wt% to 50 wt% of said of said metal compound MC, in particular of said rare earth oxycarbonate REOC, to 50 wt% to 20 wt% of said poly ionic liquid PIL.

In some embodiments, the ratio of said metal compound MC, in particular said rare earth compound REC, more particularly said rare earth oxycarbonate REOC, to said poly ionic liquid PIL is in the range of 50 wt% to 20 wt% of said of said metal compound MC, in particular of said rare earth oxycarbonate REOC, to 50 wt% to 80 wt% of said poly ionic liquid PIL.

In some embodiments, the ratio of said metal compound MC, in particular said rare earth compound REC, more particularly said rare earth oxycarbonate REOC, to said poly ionic liquid PIL is in the range of 80 wt% to 60 wt% of said of said metal compound MC, in particular of said rare earth oxycarbonate REOC, to 40 wt% to 20 wt% of said poly ionic liquid PIL.

In some embodiments, the ratio of said metal compound MC, in particular said rare earth compound REC, more particularly said rare earth oxycarbonate REOC, to said poly ionic liquid PIL is in the range of 40 wt% to 20 wt% of said of said metal compound MC, in particular of said rare earth oxycarbonate REOC, to 60 wt% to 80 wt% of said poly ionic liquid PIL.

In some embodiments, the ratio of said metal compound MC, in particular said rare earth compound REC, more particularly said rare earth oxycarbonate REOC, to said poly ionic liquid PIL is essentially 70 wt% of said of said metal compound MC, in particular of said rare earth oxycarbonate REOC, to 30 wt% of said poly ionic liquid PIL.

In some embodiments, said poly ionic liquid PIL comprises a porous structure, in particular a mesoporous structure.

In some embodiments, said poly ionic liquid PIL comprises a porosity in the range of 2 to 50 nm, in particular in the range of 2 to 20 nm.

Mesoporous networks of PIL show significantly faster CO₂ adsorption than its bulk counterparts. The outstanding CO₂ sorption in combination with a mesoporous structure will be the most appropriate for CO₂ sensing.

In some embodiments, said metal compound MC, in particular said rare earth compound REC, more particularly said rare earth oxycarbonate REOC comprises particles with a diameter in the range smaller than 200 nm, in particular smaller than 100 nm, more particularly smaller than 20 nm. This is because the smaller the size of nanoparticle, the higher is the surface area of interaction between REOC and PIL.

In some embodiments, the composition comprises additionally nanoparticles of a further metal compound MC, in particular a further rare earth compound REC, more particularly a further rare earth oxycarbonate REOC, wherein the further metal compound MC, in particular the further rare earth compound REC, more particularly the further rare earth oxycarbonate REOC comprise the same properties as discussed above concerning the first aspect of the invention.

A second aspect of the invention relates to a suspension or solution comprising a composition according to the first aspect of the invention.

In some embodiments, said metal compound MC, in particular said rare earth compound REC, more particularly said rare earth oxycarbonate REOC, and said poly ionic liquid PIL are present in the suspension or solution in form of separate particles consisting of said metal compound MC, in particular said rare earth compound REC, more particularly said rare earth oxycarbonate REOC, or said poly ionic liquid PIL

In some embodiments, said metal compound MC, in particular said rare earth compound REC, more particularly said rare earth oxycarbonate REOC, and said poly ionic liquid PIL are present in the suspension or solution in form of particles consisting of a metal compound MC center, in particular a rare earth compound REC center, more particularly a rare earth oxycarbonate REOC center, and a poly ionic liquid PIL casing, which may partially or completely enclose the center, providing mixed particles in the suspension or solution.

In some embodiments, said metal compound MC, in particular said rare earth compound REC, more particularly said rare earth oxycarbonate REOC, of the suspension or solution comprise a diameter in the range of 2 to 200 nm, in particular in the range of 2 to 100 nm, more particularly in the range of 2 to 50 nm, further more in the range of 2 to 20 nm, and said poly ionic liquid PIL particles of the suspension or solution
- comprise a diameter in the range of 2 to 500 nm, in particular in the range of 2 to 100 nm, more particularly in the range of 2 to 50 nm or
- are partially dissolved in the solvent of the solution or suspension or
- completely dissolved in the solvent of the solution or suspension.

In some embodiments, said metal compound MC, in particular said rare earth compound REC, more particularly said rare earth oxycarbonate REOC, of the suspension or solution comprises a diameter in the range of 2 to 20 nm, and said poly ionic liquid PIL particles are completely dissolved in the solvent of the solution or suspension. In order avoid clogging, the smaller particles are favorable.

In some embodiments, the suspension or solution comprises an organic solvent, water or a mixture thereof, wherein the organic solvent is selected from alcohols, ketones, acetonitrile or a mixture thereof, wherein in particular said solvent is selected from water, ethanol, acetone, isopropanol, acetonitrile or a mixture thereof.

In some embodiments, the suspension or solution comprises a viscosity of 1 to 25 mpascal*s.

A third aspect of the invention relates to a substrate comprising a composition according the first aspect of the invention, wherein on said substrate said metal compound MC, in particular said rare earth compound REC, more particularly said rare earth oxycarbonate REOC, and said poly ionic liquid PIL are deployed, wherein in particular said substrate is selected from a rigid material, such as ceramic, aluminum, sapphire, silicium or glass or a flexible material, such as polyamide or polyurethane.

In some embodiments, said substrate comprises a mixture of said metal compound MC, in particular said rare earth compound REC, more particularly said rare earth oxycarbonate REOC, and said poly ionic liquid PIL (see Fig. 1).

In some embodiments, said substrate comprises a layer of said metal compound MC, in particular a layer of said rare earth compound REC, more particularly a layer of said rare earth oxycarbonate REOC, and a layer of said poly ionic liquid PIL deposited on said a layer of metal compound MC, in particular said a layer of rare earth compound REC, more particularly said a layer of rare earth oxycarbonate REOC, layer.

In some embodiments, said substrate comprises a layer of said poly ionic liquid PIL and a layer of said metal compound MC, in particular a layer of said rare earth compound REC, more particularly a layer of said rare earth oxycarbonate REOC, deposited on said poly ionic liquid PIL layer.

A fourth aspect of the invention relates to a use of the composition according to the first aspect of the invention or a substrate according to the third aspect of the invention for CO₂ detection.

A fifth aspect of the invention relates to a use of the composition according to the first aspect of the invention or a substrate according to the third aspect of the invention in a CO₂ sensor.

The composites bring to bear the affinity of PIL and MC to interact with CO₂. Nevertheless, the main advantage is that interaction of PIL with MC dramatically changes the electrical properties of composites. This is decisive for the transduction of chemical information into electrical signal and thus for the application as a CO₂ sensor.

A sixth aspect of the invention relates to a use of the suspension or solution according to the second aspect of the invention in screen printing, drop casting, tape casting, spin casting or ink-jet printing methods.

A seventh aspect of the invention relates to a use of the suspension or solution according to the second aspect of the invention in screen printing, drop casting, tape casting, spin casting or ink-jet printing methods for manufacturing a CO₂-sensor.

The prepared solution or suspensions can be used in any of the state-of-art film fabrication method for example screen printing, drop casting, tape casting, spin casting or ink-jet printing methods. The optimization will include mainly adjusting the viscosity and/or composition of the suspension and solution. Other advantages highlighting the versatility of the composites are their high thermal stability (up to 200°C) and selective solubility. This makes them compatible with for instance MEMS processing.

An eight aspect of the invention relates to a method for CO₂ detection comprising a CO₂ sensor according to the seventh aspect of the invention.

The method comprises the steps of:
- provision of a CO₂ sensor comprising a composition according to any one of the claims 1 to 7 or a substrate according to any one of the claims 8 to 12,
- contacting said sensor with CO₂, which is to be detected, in particular contacting said sensor with CO₂, which is to be detected, at a temperature in the range of 20°C and 200°C, more particularly in the range of 20°C to 100°C, more particularly in the range of 20°C to 35°C
- chemical interaction of said CO₂ with said composition or said substrate comprising said composition providing an electrical signal based on said chemical interaction and
- analyzing said signal.

For details, see section CO₂-Detection.

### Methods of synthesis

### General methods and instruments:

PXRD X-ray powder diffraction (PXRD) patterns were registered on a PANalytical Empyrean powder diffractometer in reflection mode using Cu Kα radiation and operating at 45 kV and 40 mA. For the in-situ PXRD experiments, the Anton Paar high temperatures chamber was used.

Scanning electron microscopy (SEM) images were taken on a LEO 1530 Gemini microscope equipped with an in-lens detector at an acceleration voltage of 3 kV. For the SEM imaging, samples were coated with a 5 nm layer of sputtered Pt to prevent charging.

EDX was measured on a Hitachi SU-70 operated at 20 kV acceleration voltage. An X-Max detector (semiconductor-type, Oxford Instruments) was used.

Thermogravimetric analysis (TGA) of La(OH)₃ was conducted using a Mettler Toledo TGA/SDTA 851 e instrument under air. The heating rate was 10 K/min.

Transmission electron microscopy (TEM) images were recorded using a Philips Tecnai operating at an acceleration voltage of 300 kV. The samples were dispersed in acetone by sonication and a drop of the suspension was air-dried onto a carbon-coated copper grid.

Surface area of the nanoparticles was determined by Brunauer-Emmett-Teller (BET) nitrogen adsorption. The powders were first outgassed for 22 h at 150 °C and then the adsorption isotherm was recorded on a Quantachrome Autosorb iQ station.

### Two step synthesis of rare-earth oxycarbonates:

The reaction pathway to La₂O₂CO₃ is depicted in Scheme 1.

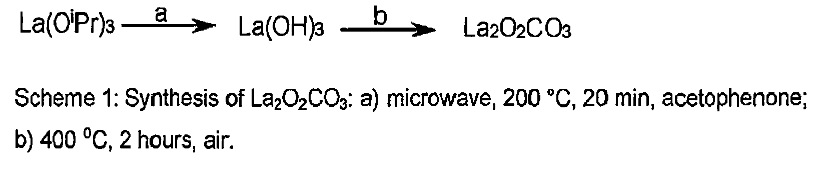

### Synthesis of La(OH)₃ nanoparticles

La(OH)₃ nanoparticles were synthesized via a modification of a previously published method (Rare earth oxycarbonates as a material class for chemoresistive CO2 gas sensors, A. Haensch, D. Koziej, M. Niederberger, N. Barsan, U. Weimar, Procedia Engineering, 5 (2010) 139-142). This modification allows for significant decreases of nanoparticles size to approximately 10 nm. Additionally, a further decrease of nanoparticles size dependent on the reaction time is feasible. In a typical synthesis 0.5 mmol (158.1 mg) of La(OiPr)₃ were dissolved in 40 mmol (4.67 mL) of acetophenone, mixed under argon atmosphere in a 10 mL pressure vial and stirred for 10 min. The brown, opaque solution was sealed and heated in CEM Explorer microwave oven at 200 °C for 20 min. Subsequently, the precipitate was extracted by centrifugation and washed three times with acetone. The nanoparticles were dried in a lab furnace at 60 °C overnight (see step a of Scheme 1).

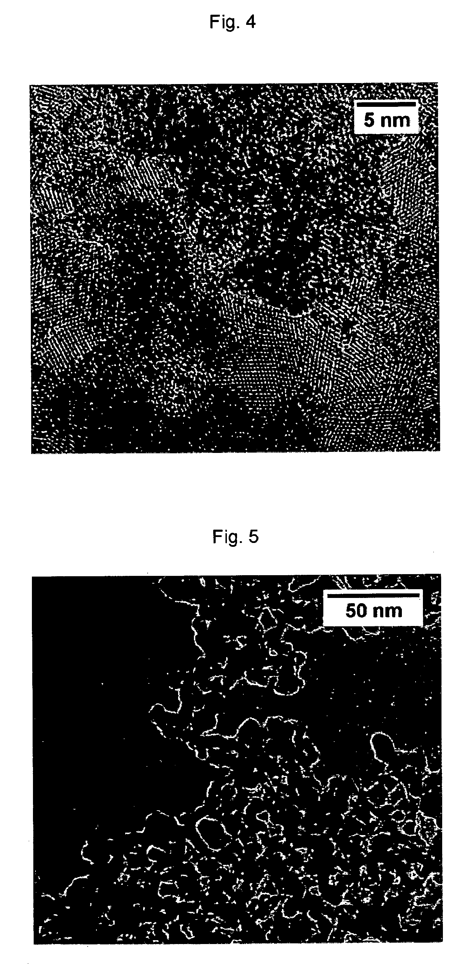

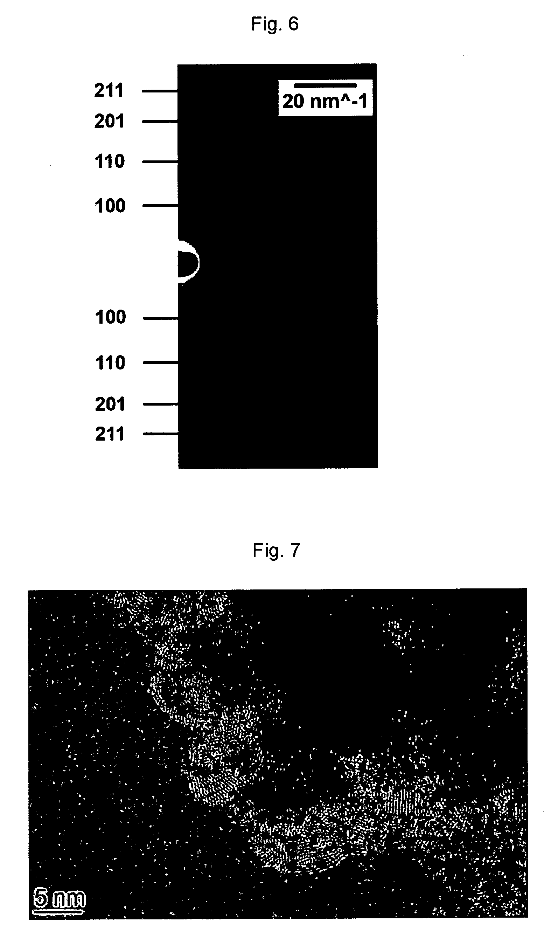

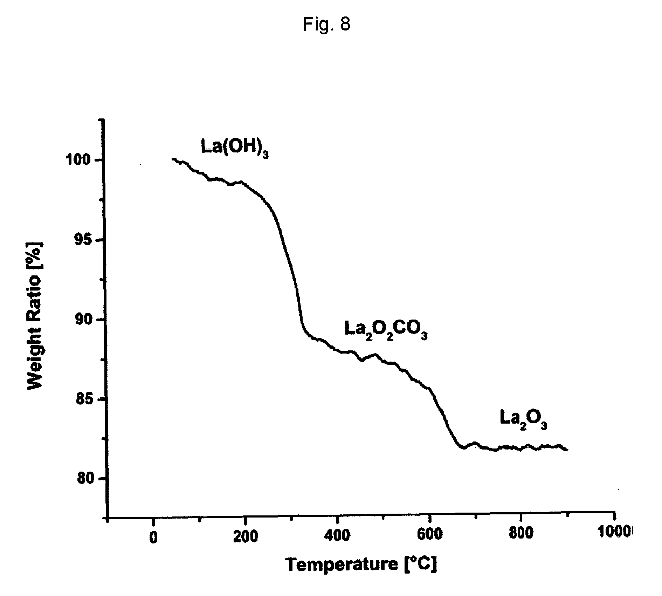

The La(OH)₃ particles and La₂O₂CO₃ particles were examined with PXRD, BET and TEM (see Fig. 4-7). The La(OH)₃ particles show a diameter of 9.3 nm and a surface area of 147.2 m²/g. The La₂O₂CO₃ particles show a diameter of 10.4 nm and a surface area of 114.3 m²/g. The thermal transformation from La(OH)₃ to La₂O₂CO₃ was investigated by TGA and in-situ PXRD (see Fig. 8, 9 and step b of Scheme 1). 400°C is the temperature needed for transformation of La(OH)₃ to La₂O₂CO₃. Lanthanum oxides may be produced by applying a higher temperature in the thermal transformation step (see Fig. 8).

### One step synthesis of rare-earth oxycarbonates:

Direct synthesis of rare-earth carbonates and oxycarbonates in CO₂ enriched solution.

In order to directly synthesize oxycarbonates nanoparticles in the solution a carbonate source has to be added to the reaction solution, for instance carbon dioxide, alkali carbonates and/or rare-earth precursors including R-CO₂⁻. In such a carbon dioxide enriched condition the direct nucleation of carbonates nanoparticles can be additionally induced by high pressure or temperature.

### In-situ synthesis of rare-earth nanoparticles on PIL:

The PILs particles were dispersed in a non-aqueous solvent. The lanthanum precursor, here lanthanum isopropoxide was dissolved in acetophenone. The reaction solution was heated in the microwave to 200 °C for 15 minutes. PILs due to their high microwave absorptivity, act as hot spots for nucleation. Thus the nanoparticles nucleate and grow directly at the surface of PI L as shown in Figure 10. The nucleation of nanoparticles is restricted only to the surface of PILs. Therefore in SEM image no lose nanoparticles, not attached to PIL, can be observed.

### In-situ polymerization of PIL on REOC nanoparticles:

The REOC nanoparticles can be dispersed in dimethyl form amide (DMF), or any other solvent used in polymerization of ionic liquids monomer, for instance [VBTMA][PF₆]. In-situ polymerization will induce grafting of the PIL at the surface or REOC and will reinforce the synergistic interaction between PIL and REOC.

### Ex-situ fabrication:

A schematic drawing of the sensor fabrication process is depicted in Fig. 2.

La₂O₂CO₃ nanoparticles and P[VBTMA][PF₆] are blended in acetonitrile and dip coated on the substrate equipped with electrodes. As-prepared sensors were dried in the oven at 80 °C for 24 hours. Different concentrations of PIULOC were tested. The optimal film consist of 77 wt% La₂O₂CO₃ (LOC) and 23 wt% P[VBTMA][PF₆] (PIL).

The impedance spectroscopy was utilized to determine the optimal composition of the sensing film. The main selection criterion is the low resistivity of the layers at 50 Hz. The impedance spectra of the optimized films, which were fabricated in the aforementioned process, are shown in Fig. 15 for comparison the impedance spectra of single components are shown. Since the impedance depends on the geometry of the films (thickness, porosity), geometry of electrodes of the film etc. the optimal compositions has to be determined dependent on the film fabrication process.

An alternative fabrication process is depicted in Fig. 3.

La(OH)₃ (ICDD File Card No. 06-0585) nanoparticles produced by microwave synthesis are dispersed in an organic matrix following a procedure documented in literature (S. K. Biswas, J.-O. Baeg, S.-J. Moon, K. Kong, W.-W. So, J. Nanopart. Res. 2012, 14, 667), which is then spread on the sensor substrate by the doctor blade approach. The layer was set to be 100 µm thick. A thermal treatment step at 400°C followed. This allowed the removal of the organics in the dispersion as well as the transformation of the La(OH)₃ to La₂O₂CO₃ (ICDD File Card No. 48-1113). The resulting film had a thickness of approximately 2 µm with a porosity of 31 %. Alternatively, La₂O₂CO₃ particles can be directly used. Finally, the polymer layer is applied. The film consists of 52 wt% La₂O₂CO₃ (REOC) and 48 wt% P[VBTMA][PF₆] (PIL).

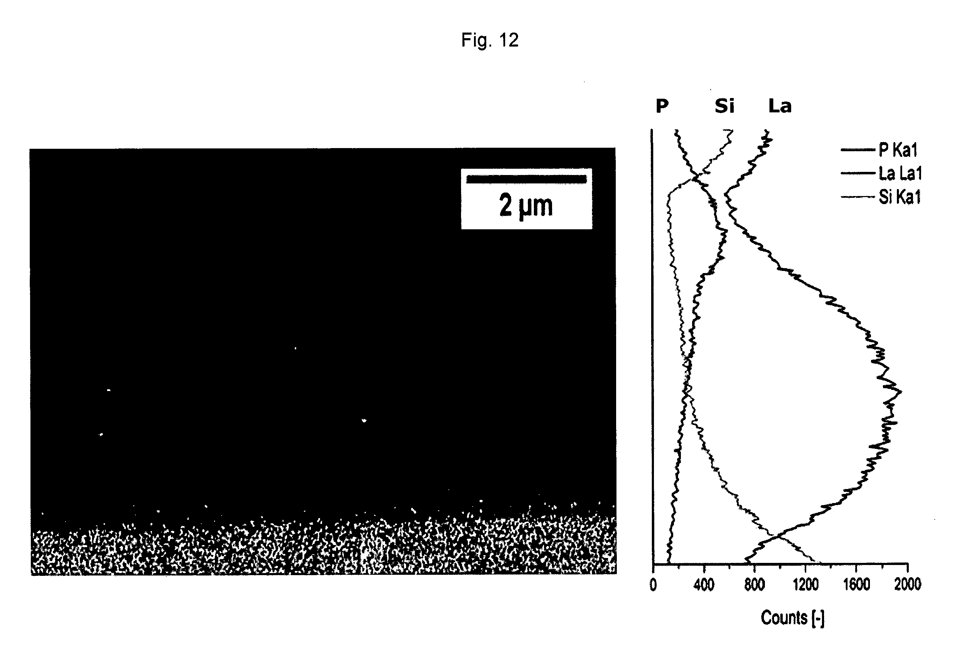

A SEM picture of a cross section the La₂O₂CO₃/P[VBTMA][PF₆] film is depicted in Fig. 11. A corresponding EDX map of a cross-section of the La₂O₂CO₃/P[VBTMA][PF₆] film and concentration profile recorded for La, Si and P elements is depicted in Fig. 12. The La₂O₂CO₃/P[VBTMA][PF₆] film shows a homogenous distribution of P[VBTMA][PF₆] in the La₂O₂CO₃ film

The same methods apply for other metal compounds or rare earth compounds or PIL discussed above.

### Characterisation:

### Layers of the composition:

The La(OH)₃ nanoparticles were characterized using x-ray diffraction (XRD) spectroscopy as well as transmission electron microscopy (TEM). According to size calculations in XRD, the particle size is in the 9-11 nm range and is confirmed by TEM images (see Fig. 4 and 5). The degree of crystallinity seems to be very high.

Thermogravimetric analysis (TGA) was used to find the ideal temperature for the La(OH)₃ to La₂O₂CO₃ transformation (see Fig. 8). An in-situ XRD analysis concerning the conversion of La(OH)₃ to La₂O₂CO₃ and La₂O₃ is depicted in Fig. 9. Scanning electron microscopy (SEM) was found to be the most appropriate method for verifying the quality of the films produced. In Fig. 10, an SEM image of the cross section of a typical film produced using the aforementioned approach of Fig. 3 is depicted.

FTIR measurements were performed to investigate the interaction between the PIL (P[VBTMA][PF₆]) and LOC (lanthanum oxycarbonate) nanoparticles, as shown in Figure 20. LOC nanoparticles were first dispersed in PIL-acetonitrile solution and then centrifuged. As centrifuge powders were washed several times in acetonitrile to remove all PIL, which was not firmly grafted at the surface. Presence of PIL specific vibration at the surface of LOC suggests that PIL is indeed irreversibly grafts at the surface of LOC. This strong chemical interaction is likely responsible for the synergistic effect on the electrical properties of the films and their sensitivity to CO₂.

### CO₂ detection:

Sensitivity towards CO₂ and cross-sensitivity to CO in humid air were tested in a continuous-flow chamber. A computer-controlled gas mixing system equipped in Bronkhorst mass flow controllers adjusts the gas concentrations at the constant flow of 200 mL min⁻¹ in air as a carrier gas. To monitor changes in the DC resistance or in the AC impedance of the sensor during exposure to the target gases, the electrodes of the sensor were connected to a Keithley 617 electrometer or Zahner IM6 impedance analyzer, respectively.

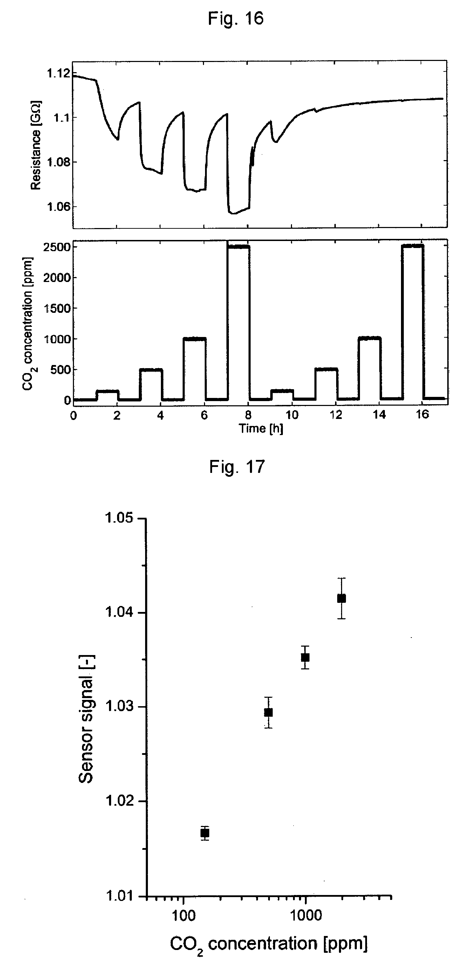

To characterise the CO₂ sensing performance, the sensors are subjected to different concentrations of CO₂ and the resistance of the films is measured at the same time. In Fig. 16, a typical resistance changes profile and in Figure 17 corresponding sensor signal are shown. The first curve (top) shows the evolution of the resistance with time whereas the second curve (bottom) depicts the concentration changes of CO₂ versus time in hours. Pulses of 150, 500, 1000 and 2500 ppm were performed. The first four CO₂ pulse led to a reduction in sensor resistance. After 8 hours, the channel for CO₂ was inverted so that synthetic air would flow through instead. Such a blank experiment allows demonstrating that the measured signals are indeed from CO₂ and not an artefact related to the gas mixing system. However, small peaks appear at the first two pulses 150 and 500 ppm pulses. These are due to some residual CO₂ in the gas tubes.

To demonstrate sensors' selectivity, the sensors are subjected to different concentration of CO₂ and CO as shown in Figure 14. The first curve (top) shows resistance changes of a sensor upon exposure to 250, 500, 1000 and 2000 ppm of CO₂ and 10, 20 and 50 ppm CO. The CO₂ pulses lead to significant decreases of the resistance, whereas CO pulses of did not have a significant impact on the resistance. The second curve (bottom) shows resistance changes of the commercial metal-oxide-based sensor, which was simultaneously used. Such a reference sensor, as expected, shows reversed trend, namely high sensitivity to CO and lack of sensitivity to CO₂.

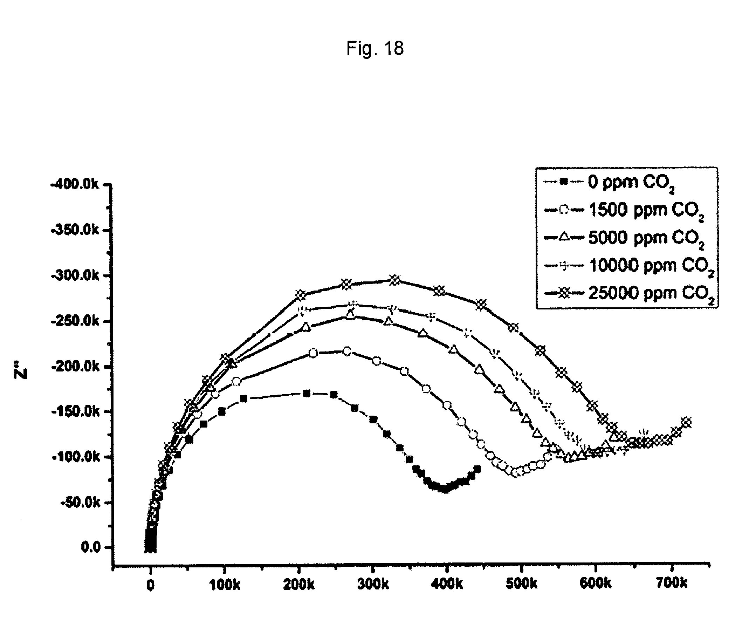

In addition to DC resistance (static resistance) measurements, electrochemical impedance spectroscopy (EIS) was performed. In Fig. 18, the impedance of the sensor at various CO₂ content is plotted in a Nyquist plot. With increasing CO₂ concentration, the semi-circles become larger, indicating an increasing sensor signal. In Fig. 19 the model circuit of the system is shown. The resistor simulates the charge transfer resistance, the Warburg impedance shows diffusion and becomes important only at low frequencies, finally, the constant phase element (CPE) is the counterpart for the double-layer capacitance. Furthermore, AC impedance measurements show that capacitive readout of sensor signal is also possible.

### Short description of the figures:

- Fig. 1: shows a structure of composite materials: nanoparticles of the metal compound (dots marked with arrows) and mesoporous PIL composite materials (solid block with transparent pores). In an alternative, the PIL composite materials may be distributed in a similar way as the marked dots.
- Fig. 2: shows a one step sensor fabrication on a substrate equipped with electrodes. A dispersion of La₂O₂CO₃ nanoparticles and PIL in volatile solvent is deposited on the substrate via dip coating.
- Fig. 3: shows a three step sensor fabrication on a substrate equipped with electrodes. First, a dispersion of La(OH)₃ nanoparticles in an organic matrix is spread on the substrate via doctor blade (i). Following, a calcination step is performed to remove the organic matrix and to transform the La(OH)₃ into La₂O₂CO₃ (ii). Finally, the polymer layer (P[VBTMA][PF₆]) is applied (iii).
- Fig. 4: shows a HR-TEM image of La(OH)₃ nanoparticles.
- Fig. 5: shows a TEM image of La(OH)₃ nanoparticles.
- Fig. 6: shows an indexed diffraction pattern of La(OH)₃ nanoparticles.
- Fig. 7: shows a HR-TEM image of La₂O₂CO₃ nanoparticles produced according to scheme 1.
- Fig. 8: shows a thermogravimetric analysis concerning the thermal conversion of La(OH)₃ to La₂O₂CO₃ and La₂O₃.
- Fig. 9: shows in-situ XRD analysis concerning the conversion of La(OH)₃ to La₂O₂CO₃ and La₂O₃.
- Fig. 10: shows SEM image of La(OH)₃ grown directly at the surface of mesoporous PIL.
- Fig. 11: shows a SEM picture of a cross-section of the La₂O₂CO₃/P[VBTMA][PF₆] film according to Fig. 3. Acceleration voltage was 3 kV, Working distance 3.6 mm and an in-lens detector was used.
- Fig. 12: shows EDX map of a cross-section of the La₂O₂CO₃/P[VBTMA][PF₆] film according to Fig. 3 and concentration profile recorded for La, Si and P elements.
- Fig. 13: shows the relative changes of resistance of a CO₂ sensor versus time. The film composition was 77% LOC (lanthanum oxycarbonate) and 23% PIL (P[VBTMA][PF₆]). The sensor is subjected to different concentrations of CO₂ (150, 250, 500, 1000, 1500, and 2000). In the inset, sensor signal vs. concentration of CO₂ is shown.
- Fig. 14: shows the absolute changes of resistance of a CO₂ sensor versus time. The film composition was 77% LOC (lanthanum oxycarbonate) and 23% PIL (P[VBTMA][PF₆]). The sensor is subjected to different concentrations of CO₂ (250, 500, 1000, and 2000) and of CO (10, 20, 50 ppm). For comparison to the signal of conventional MOX sensors is shown at the bottom.
- Fig. 15: shows a Nyquist plot of impedance spectra of LOC (lanthanum oxycarbonate), PIL (P[VBTMA][PF₆]) and composites of thereof. The film composition was 77% LOC and 23% PIL.
- Fig. 16: shows the change of resistance of a CO₂ sensor versus time. The film composition was 52% LOC (lanthanum oxycarbonate) and 48 % PIL (P[VBTMA][PF₆]). The sensor is subjected to different concentrations of CO₂ (150, 500, 1000 and 2500). In the second part, synthetic air was fed instead of CO₂. A slight decrease of resistance is due to residual CO₂ in the tubes. The humidity level was 50% relative humidity.
- Fig. 17: shows the sensor signal sensor versus time. The film composition was 52% LOC (lanthanum oxycarbonate) and 48 % PIL (P[VBTMA][PF₆]). The sensor is subjected to different concentrations of CO₂ (150, 500, 1000 and 2500). The humidity level was 50% relative humidity.
- Fig. 18: shows a Nyquist plot of impedance spectra at various CO₂ content at 50% relative measured at the frequency range between 50 Hz and 2 MHz. The film composition was 52% LOC and 48 % PIL.
- Fig. 19: shows an equivalent circuit modelling the sensor. The resistance stands for charge transfer resistance, the Warburg for diffusion.
- Fig. 20: FTIR spectra of PIL (P[VBTMA][PF₆]), LOC and PIL grafted at the surface of LOC. The presence of PIL at the surface of LOC nanoparticles, even after thorough washing in acetonitrile proofs that PIL is irreversible grafted at the surface of LOC. Thus, there is a strong interaction between the surface of LOC nanoparticles and PIL.

## Claims

1. A composition, in particular for CO₂ detection, comprising at least one metal compound (MC) and a poly ionic liquid (PIL).

2. The composition according to claims 1, wherein the metal of said the metal compound (MC) is selected from rare earth metals, metals from the group of lanthanides or metals from the group of actinoides and/or the metal compound (MC) is selected from a metal hydroxide, metal carbonate, metal oxide or metal oxycarbonate or mixture thereof, in particular from a metal oxide or metal oxycarbonate, more particularly said metal compound (MC) is a metal oxycarbonate.

3. The composition according to any one of the claims 1 or 2, wherein said metal compound (MC) is a rare earth oxycarbonate (REOC), wherein in particular the rare earth metal of said rare earth oxycarbonate (REOC) is selected from lanthanum, cerium, neodymium, praseodymium or gadolinium, in particular from lanthanum, cerium, neodymium or praseodymium, more particularly the rare earth metal of said rare earth oxycarbonate (REOC) is lanthanum.

4. The composition according to claim 1, wherein said poly ionic liquid (PIL) comprises a polymer structure of ionic liquid monomers, wherein in particular said poly ionic liquid (PIL) comprises a reversible CO₂ capturing capacity.

5. The composition according to any one of the claims 1 or 4, wherein said poly ionic liquid (PIL) is selected from polymers of pyrrolidinium based ionic liquids, sulfonium based ionic liquids, alanine based ionic liquids, choline based ionic liquids, acetonitrile based ionic liquids, ammonium based ionic liquids, pyridinium based ionic liquids, imidazolium based ionic liquids or phosphonium based ionic liquids, in particular of ammonium-based ionic liquids, pyridinium-based ionic liquids, imidazolium-based ionic liquids or phosphonium-based ionic liquids, more particularly said poly ionic liquid (PIL) is selected from (p-vinylbenzyl)triethyl ammonium tetrafluoroborate P[VBTEA][BF₄], 1-(p-vinylbenzyl)pyridinium tetrafluoroborate P[VBP][BF₄], (p-vinylbenzyl)trimethyl ammonium hexafluorophosphate P[VBTMA][PF₆], 2-(methacryloyloxy) ethyltrimethylamnonium tetrafluoroborate P[MATMA][BF₄], (p-vinylbenzyl)trimethyl ammonium trifluoromethane sulfonamide P[VBTMA][Tf₂N]; p-vinylbenzyl)trimethyl ammonium tetrafluoroborate P[VBTMA][BF₄], 1-(p-vinylbenzyl)-3-methyl-imidazolium tetrafluoroborate P[VBMI][BF₄], (p-vinyl benzyl)tributylammonium tetrafluoroborate P[VBTBA][BF₄] or p-vinylbenzyl)triethyl phosphonium tetrafluoroborate P[VBTEP][BF₄], more particularly said poly ionic liquid (PIL) is (p-vinylbenzyl)trimethyl ammonium hexafluorophosphate P[VBTMA][PF₆]

6. The composition according to any one of the previous claims, wherein the ratio of said at least one metal compound (MC), in particular said at least one rare earth compound (REC), more particularly said at least one rare earth oxycarbonate (REOC), to said poly ionic liquid (PIL) is in the range of 1 wt% to 99 wt% of said of said metal compound (MC), in particular of said rare earth compound REC, more particularly of said rare earth oxycarbonate (REOC), to 99 wt% to 1 wt% of said poly ionic liquid (PIL).

7. The composition according to any one of the previous claims, wherein said metal compound (MC), in particular said rare earth compound (REC), more particularly said rare earth oxycarbonate (REOC) comprises particles with a diameter in the range smaller than 200 nm, in particular smaller than 100 nm, more particularly smaller than 20 nm.

8. A suspension or solution comprising a composition according to any one of the claims 1 to 7.

9. The suspension or solution according to claim 8, wherein said metal compound (MC), in particular said rare earth compound (REC), more particularly said rare earth oxycarbonate (REOC), and said poly ionic liquid (PIL) are present
- in form of separate particles consisting of said metal compound (MC), in particular said rare earth compound (REC), more particularly said rare earth oxycarbonate (REOC), and of said poly ionic liquid (PIL), or
- in form of particles consisting of a metal compound (MC) center, in particular a rare earth compound (REC) center, more particularly a rare earth oxycarbonate (REOC) center, and a poly ionic liquid (PIL) casing, which may partially or completely enclose the center, providing mixed particles.

10. The suspension or solution according to any one of the claims 8 or 9, wherein said metal compound (MC), in particular said rare earth compound (REC), more particularly said rare earth oxycarbonate (REOC), particles comprise a diameter in the range of 2 to 200 nm, in particular in the range of 2 to 100 nm, more particularly in the range of 2 to 20 nm and said poly ionic liquid (PIL) particles comprise a diameter in the range of 2 to 500 nm, in particular in the range of 2 to 100 nm, more particularly in the range of 2 to 20 nm or said poly ionic liquid (PIL) particles are partially or completely dissolved in the solvent of the solution or suspension.

11. A substrate comprising a composition according to any one of the claims 1 to 7, wherein on said substrate said metal compound (MC), in particular said rare earth compound (REC), more particularly said rare earth oxycarbonate (REOC), and said poly ionic liquid (PIL) are deployed, wherein in particular said substrate is selected from a rigid material, such as ceramic, aluminium, sapphire, silicium or glass or a flexible material, such as polyamide or polyurethane.

12. The substrate according to claim 11, wherein said substrate comprises
- a mixture of said metal compound (MC), in particular said rare earth compound (REC), more particularly said rare earth oxycarbonate (REOC), and said poly ionic liquid (PIL), or
- a layer of said metal compound (MC), in particular a layer of said rare earth compound (REC), more particularly a layer of said rare earth oxycarbonate (REOC), and a layer of said poly ionic liquid (PIL) deposited on said layer of said metal compound (MC), in particular said layer of said rare earth compound (REC), more particularly said layer of said rare earth oxycarbonate (REOC), layer or
- a layer of said poly ionic liquid (PIL) and a layer of said metal compound (MC), in particular a layer of said rare earth compound (REC), more particularly a layer of said rare earth oxycarbonate (REOC), deposited on said layer of said poly ionic liquid (PIL) layer.

13. Use of the composition according to any one of the claims 1 to 7 or a substrate according to any one of the claims 11 to 12 for CO₂ detection or in a CO₂ sensor.

14. Use of the suspension or solution according to any one of the claims 8 to 10 in screen printing, drop casting, tape casting, spin casting or ink-jet printing methods, in particular in screen printing, drop casting, tape casting, spin casting or ink-jet printing methods for manufacturing a CO₂ sensor.

15. A CO₂ sensor comprising composition according to any one of the claims 1 to 7 or a substrate according to any one of the claims 11 to 12 or a CO₂ sensor manufactured by use of a suspension or solution according to any one of the claims 8 to 10.
